# EUROPEAN PATENT APPLICATION

(11) **EP 3 254 639 A1**
(43) Date of publication of application: **13.12.2017**
(21) Application number: 16203547.1
(22) Date of filing: 12.12.2016
(51) Int. Cl.: A61B 90/00, A61B 6/00, A61B 17/00

(54) **A SURGICAL LOCALIZING SCALE**

(30) Priority: 08.06.2016 CN 201620554957 U
(71) Applicant: Tinavi Medical Technologies Co., Ltd., Beijing 100192 (CN)
(72) Inventor: XU, Jin, Beijing, Beijing 100192 (CN); LIU, Yajun, Beijing, Beijing 100192 (CN); TIAN, Wei, Beijing, Beijing 100192 (CN); ZHANG, Weijun, Beijing, Beijing 100192 (CN); WANG, Binbin, Beijing, Beijing 100192 (CN); FENG, Yun, Beijing, Beijing 100192 (CN)
(74) Representative: ABG Patentes, S.L.

(57) **Abstract**

The present utility model relates to a surgical positioning scale, including a scale face or two scale faces having a normal angle of 2° to 10° therebetween, an edge of the scale face(s) being connected to one end of a scale handle, and another end of the scale handle being provided with a connector for connecting with a mechanical arm of a surgical robot; and marker points being disposed on the scale face(s), and the total number of the marker points being at least four. Because of using a single scale face or two scale faces that are almost coplanar, the present utility model has a small volume, and can be placed immediately close to the surface of a human body, and can ensure that a 3D image reconstructed in CBCT includes all marker points. In addition, any two of the marker points have unequal distances therebetween, which enables the marker points on the same scale face to have the feature of anisotropy, so that accuracy rate of identification of marker points can be increased when the present utility model is applied to an auto-registration algorithm of a 3D X-ray image, thereby ensuring the positioning of the system more accurate.

## Description

### TECHNICAL FIELD

The present utility model relates to a medical instrument, and in particular, to a surgical positioning scale/ localizing ruler used for surgical navigation and localization.

### BACKGROUND

Generally, in various surgical navigation systems, and in particular, in a robotic surgery system, an auto-registration algorithm is one of the core algorithms, a positioning scale or positioning ruler is a reference standard when this algorithm is implemented and is a key component that affects localizing or positioning precision in surgery. When an image is captured, the positioning scale is generally placed in the scanning field of view (FOV) of an imaging device and is scanned by the imaging device to form a three-dimensional (3D) medical X-ray image. Then, marker points specifically distributed on the positioning scale are presented on the image. Spatial localization calculation can be performed according to distribution of the specific marker points in the 3D X-ray image, so that the auto-registration between the image space and the robot space can be implemented so as to perform planning and navigation of surgery.

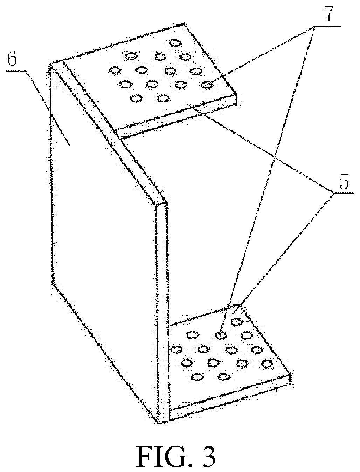

The patent application entitled "METHOD FOR ADAPTING C-ARM SYSTEM TO PROVIDE THREE-DIMENSIONAL IMAGING INFORMATION", published as CN103961130A describes a 3D target containing X-ray opaque marker balls for implementing spatial localization, and a structure thereof, as shown in FIG. 3, includes two positioning faces 5 that are spaced and disposed in parallel and a connecting face 6 for connecting the two positioning faces 5. Each of the positioning faces 5 is provided with regularly distributed marker points 7, so that spatial localization can be performed by means of the known three-dimensional arrangement of the X-ray opaque marker balls.

However, when a 3D medical X-ray image is captured, and in particular, when an image is captured by Cone beam CT (CBCT), the image capturing process is a continuous scanning process performed around the positioning scale. But the effective imaging FOV of the CBCT is relatively small, so the positioning scale needs to be as small as possible and be able to keep close to the surface of a human body. At this time, if a conventional positioning tool is used, a situation, such as incomplete scanning or loss of imaging, may occur in a rotary scanning process of the CBCT. In addition, because accurate computerized automatic identification needs to be performed for marker points in a 3D reconstruction image, distribution of the marker points needs to be anisotropic. However, marker points 7 on the conventional positioning tool are relatively regular and do not have anisotropy. Therefore, when the conventional positioning tool is applied to an auto-registration algorithm of a 3D X-ray image, complexity of identifying marker points is increased, and a correct identification rate of the marker points is reduced, thereby affecting the positioning accuracy of the system.

### SUMMARY

Aiming at the above problems, the present utility model seeks to provide a new CBCT image-based surgical positioning scale.

In order to achieve the above goal, the following technical solution is used in the present utility model: a surgical positioning scale, characterized that including: a scale face or two scale faces with a normal angle of 2° to 10° therebetween, an edge of the scale face(s) being connected with one end of a scale handle, and another end of the scale handle being provided with a connector for connecting with a mechanical arm of a surgical robot; and marker points being disposed on the scale face(s), and the total number of the marker points being at least four.

Any two of the marker points have unequal distances therebetween.

The scale face(s) and the scale handle are all made of an X-ray transparent material, and the marker points are made of an X-ray opaque material.

Due to employing the above technical solution, the present utility model has the following advantages: 1. the present utility model has a small volume, it can be placed immediately close to the surface of a human body, and can ensure that the reconstructed 3D image of the CBCT contains all marker points; 2. in the present utility model, distances between any two of the marker points are unequal, which enables the marker points on the same scale face to be anisotropic. As a consequence the accuracy rate of identification of marker points can be increased when the present utility model is applied to an auto-registration algorithm of a 3D X-ray image, thereby ensuring positioning of the system more accurate.

### BRIEF DESCRIPTION OF THE DRAWINGS

FIG. 1 is a structural schematic diagram of Embodiment 1 of the present utility model;
FIG. 2 is a structural schematic diagram of Embodiment 2 of the present utility model;
FIG. 3 is a structural schematic diagram of a conventional surgical positioning tool.

### DETAILED DESCRIPTION

The present utility model is described in detail below with reference to the accompanying drawings and embodiments.

### Embodiment 1:

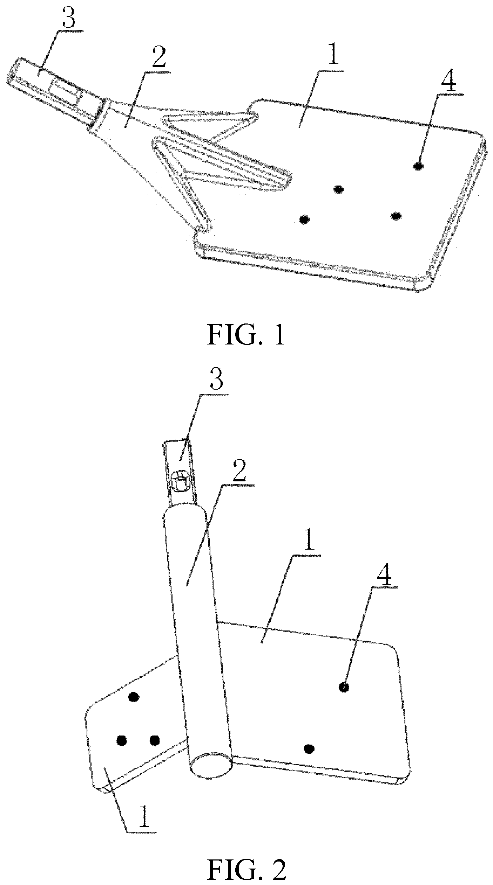

As shown in FIG. 1, this embodiment includes a scale face 1 and a scale handle 2 connected with an edge of the scale face 1, wherein a connector 3 for connecting with a mechanical arm of a surgical robot is disposed on an end of the scale handle 2, and the scale face 1 and the scale handle 2 are both made of an X-ray transparent material. The scale face 1 is provided with at least four marker points 4, and the marker points 4 are made of an X-ray opaque material.

Further, any two of the marker points 4 have unequal distances therebetween.

### Example 2:

As shown in FIG. 2, this embodiment includes two scale faces 1, a normal angle between the two scale faces 1 ranges from 2° to 10°, edges of the two scale faces 1 are both connected to a scale handle 2, a connector 3 for connecting with a mechanical arm of a surgical robot is disposed on an end of the scale handle 2, wherein the scale faces 1 and the scale handle 2 are all made of an X-ray transparent material. The two scale faces 1 are both provided with marker points 4 made of an X-ray opaque material, and the total number of the marker points 4 on the two scale faces 1 is no less than four.

Further, any two of the marker points 4 have unequal distances therebetween.

When surgical navigation is performed, a mechanical arm of a surgical robot may capture a 3D medical image by using a surgical positioning scale of the present utility model in combination with CT or CBCT: the surgical positioning scale may be mounted on an end of the mechanical arm and may be moved to an effective FOV of the CT or CBCT in a body position of a patient. When an image is being captured, the surgical positioning scale may directly face and immediately keep as close to the lesion of the observed body position as it can, so as to prevent the marker points 4 on the surgical positioning scale from being occluded or overlapped during imaging as much as possible. After completion of the image acquisition, the image is transmitted to an upper computer. The upper computer may perform spatial localization calculation according to the specific distribution of marker points of the surgical positioning scale in the 3D medical X-ray image, and finally realize auto-registration between image space and surgical robotic space , and then perform the planning and navigation of surgery.

As compared with the conventional surgical positioning scale, the present utility model has the following advantages: 1) the present utility model has a small volume so that it can be placed immediately close to the surface of a human body, and can ensure that the reconstructed 3D image of the CBCT contains all marker points; 2) distances between any two of the marker points 4 are unequal, which enables the marker points 4 on the same scale face 1 to have the feature of anisotropy. As a consequence, the accuracy rate of identification of marker points 4 can be increased when the present utility model is applied to an auto-registration algorithm of a 3D X-ray image, thereby ensuring positioning of the system more accurate.

The present model utility is merely described with the above embodiments, and structures of each component, disposition positions, and connections thereof are all able to be changed. On the basis of the technical solution of the present utility model, all modifications or equivalent variations made on a specific component according to the utility model principle of the present utility model should not be excluded from the protection scope of the present utility model.

## Claims

1. A surgical positioning scale, characterized that comprising:
a scale face or two scale faces with a normal angle of 2° to 10° therebetween, an edge of the scale face(s) being connected with one end of a scale handle, and another end of the scale handle being provided with a connector for connecting with a mechanical arm of a surgical robot; and marker points being disposed on the scale face(s), and the total number of the marker points being at least four.

2. The surgical positioning scale according to claim 1, characterized that any two of the marker points have unequal distances therebetween.

3. The surgical positioning scale according to claim 1 or 2, characterized that the scale face(s) and the scale handle are all made of an X-ray transparent material, and the marker points are made of an X-ray opaque material.
